# EUROPEAN PATENT SPECIFICATION

(11) **EP 1 973 415 B1**
(45) Date of publication and mention of the grant of the patent: **17.10.2018**
(21) Application number: 06824235.3
(22) Date of filing: 16.11.2006
(51) Int. Cl.: A23D 9/013, A23D 9/04, C11C 1/04, C11C 3/06, C11C 3/10, A01N 37/14

(54) **MODIFIED COCONUT OILS WITH BROAD ANTIMICROBIAL SPECTRUM**
MODIFIZIERTE KOKOSNUSSÖLE MIT BREITEM ANTIMIKROBIELLEM SPEKTRUM
HUILES DE NOIX DE COCO MODIFIEES A LARGE SPECTRE ANTIMICROBIEN

(30) Priority: 07.12.2005 MY 0505720
(43) Date of publication of application: 01.10.2008
(73) Proprietor: Malaysian Agricultural Research and Development Institute (MARDI), Selangor (MY)
(72) Inventor: LONG, Kamariah, Selangor (MY)
(74) Representative: Samson & Partner Patentanwälte mbB
(86) International application number: PCT/MY2006/000028
(87) International publication number: WO 2007/067028

(56) References cited:
- EP-A1- 1 588 631
- WO-A1-00/74497
- US-A- 5 208 257
- US-A- 5 378 731
- US-A- 5 569 461
- US-A- 5 639 790
- US-A1- 2002 068 014
- US-A1- 2003 129 294
- US-B1- 6 699 907
- R RODRÍGUEZ ET AL: "Hidrólisis del aceite de coco (Cocos nucífera L) mediante enzimas estereoespecíficas y sin especificidad posicional", GRASAS Y ACEITES, vol. 48, no. 1, 1 January 1997 (1997-01-01), pages 6-10, XP55034673,
- LIH-LING WANG ET AL: "Inhibition of Listeria monocytogenes by monoacylglycerols synthesized from coconut oil and milkfat by lipase-catalyzed glycerolysis", JOURNAL OF AGRICULTURAL AND FOOD CHEMISTRY, AMERICAN CHEMICAL SOCIETY, US, vol. 41, no. 6, 1 January 1993 (1993-01-01), pages 1000-1005, XP002371948, ISSN: 0021-8561, DOI: 10.1021/JF00030A033
- NANDI S ET AL: "Production of medium chain glycerides from coconut and palm kernel fatty acid distillates by lipase-catalyzed reactions", ENZYME AND MICROBIAL TECHNOLOGY, STONEHAM, MA, US, vol. 36, no. 5-6, 1 April 2005 (2005-04-01), pages 725-728, XP027765973, ISSN: 0141-0229 [retrieved on 2005-04-01]
- SUMIT NANDI ET AL: "Production of Medium Chain Glycerides and Monolaurin from Coconut Acid Oil by Lipase-Catalyzed Reactions", JOURNAL OF OLEO SCIENCE, vol. 53, no. 10, 1 January 2004 (2004-01-01), pages 497-501, XP55034667, ISSN: 1345-8957, DOI: 10.5650/jos.53.497

## Description

### Field of the Invention

This invention relates to modified oil having antimicrobial properties, in particular to modified coconut oil compositions which contain mixtures of medium chain fatty acids having 8 to 12 carbon atoms such as caprylic acid, capric acid, lauric acid and their corresponding monoglycerides. Said modified coconut oil is derived from catalytic activity of 1,3 positional specific lipase on coconut oil.

### Background of the Invention

Medium chain free fatty acids and their corresponding monoglycerides have been found to have a broad spectrum of anti microbial activity against enveloped viruses and various bacteria in vitro (Kabara, 1978; Shibasaki and Kato, 1978; Welsh et al. 1979 Thormar et al., 1987; Isaacs et al. 1995), including human pathogens like herpes simplex virus (Thormar et al. 1987; Kristmundsdottir et al. 1999), Nesseria gonorrhoeae (Bergsson et al., 1999), Candida albicans (Bergsson et al, 2001), Chlamydia trachomatis (Bergsson et al., 1998), Helicobacter pylori (Bergsson et al, 2002) and Staphylococcus aureus (Kabara 1984). In addition, these compounds also known to have antimicrobial effect against food-borne pathogens like Listeria monocytogenes (Wang and Johnson, 1992), enterotoxigenic Escherichia coli (Petschow et al 1998) and Clostridium botulinum (Glass and Johnson, 2004). The mechanism by which these lipids kill bacteria is not known, but electron microscope studies indicate that they disrupt cell membrane permeability barrier (Bergsson et al, 1998; Thormar et al. 1987).

Saturated medium chain fatty acids are fatty acids that have 8 to 12 carbon atoms (C₈ to C₁₂). Among the medium chain fatty acids, lauric acid and its corresponding monoglyceride, monolaurin, has been investigated extensively as an antimicrobial agent for foods and cosmetics (Shibasaki and Kato, 1978; Kabara, 1984). Lauric acid is a disease fighting agent that it is present in breast milk. The body converts lauric acid to a fatty acid derivative (monolaurin), which is the substance that protects infants from viral, bacterial or protozoal infections. Hierholzer and Kabara (1982) showed that monolaurin has virucidal effects on RNA and DNA viruses, which are surrounded by a lipid membrane.

The use of medium chain fatty acids and its corresponding monoester as anti- microbial compound in several applications had been patented. i.e. monolaurin have been used in cleansing and conditioning hairs as well as the coat of animals (US Patent 5,378,731). This antimicrobial shampoo composition contains monolaurin which is safe for human and animal use. Monolaurin can also be widely employed in toiletries and household articles, which need antifungal properties (US Patent 5,569,461 and 5,658,584). In addition it can be combined with bacteriocin i.e. nisin for treatment of bacterial infections of the genus *Helicobacter* that causes various gastrointestinal disease including gastritis and ulcer (US Patent 5,660,842 : US Patent 5,804,549). These fatty acids and their derivatives thereof were also claimed as a dietary supplement for controlling or reducing human's weight. (US Patent 6,054,480). Additionally, these compounds and their monoesters can be used to reduce microbial contamination in processed meat and are particularly related to products and processes to disinfect poultry carcasses. Moreover, they help to kill harmful microbes on the udder of a milk-producing animal (US Patent 6,699,907). They are also effective course of treatment for skin, mucous membrane and hair lesion (US Patent 5,208,257). The latest invention showed that these fatty acids could be therapeutic agents for treatment of Alzheimer's disease and other diseases associated with reduced neuronal metabolism, including Parkinson' disease, Huntington's disease and epilepsy (US Patent 6,835,750).

Previous works have shown that susceptibility to medium chain fatty acids varies considerably among species; certain microbes were sensitive to certain fatty acids and monoglycerides. For example, Bergsson et. al 1998 showed that lauric acid, capric acid and monocaprin caused a greater than 10,000-fold reduction in the infectivity titer. When fatty acids and their monoglycerides were further compared at low concentration and shorter exposure times, lauric acid was found to be more active than capric acid and monocaprin whereby at a concentration of 5 mM for 5 min, lauric acid caused more than 100,000 fold inactivation of *C. trachomatis* as compared to monocaprin, monolaurin and monocaprylin at 10 mM concentration, which had negligible effect on *C. trachomatis.* A work done by Bergsson et al. (2002) found that none of the medium chain fatty acids (C₈, C₁₀ and C₁₂) and their monoglycerides derivatives showed significant antibacterial activity against *Salmonella* spp and *E*. *Coli.* However, in this experiment it was found that monocaprin and monolaurin at 10 mM concentration for 30 min at 37°C proved to be the most active against *H. pylori.* Incorporation of monolaurin at 250 and 500 ppm into naturally contaminated cottage cheese resulted in more than 90% inhibition of both *Pseudomonas* spp and coliform (Bautista et al., 1993). In the case of C. *albicans,* it was found that capric acid and lauric acid caused the fastest and most effective killing as compared with their derivatives monoglycerides (Bergsson et al. 2001). However, myristic acid, palmitoleic acid, oleic acid and their derivate monoglycerides did not show antimicrobial effect.

R. Rodriguez et al., Grasas y Aceites, Vol. 48(1) (1997), p. 6-10 disclose the percentage of various fatty acids based on the total fatty acid content and the content of various monoglycerides based on the total glyceride content of coconut oil after enzymatic treatment with *Candida cyclindrica, Mucor miehei* and Lipozyme IM-20. L. Wang et al. J. Agric. Food Chem., Vol. 41 (1993), p. 1000-1005, disclose the partial hydrolysis of coconut oil with commercial lipase from *Pseudomonas fluorescens.* S. Nandi et al., Enzyme and Microbial Technology 36 (2005), 725-728), disclose the further hydrolysis of fatty acid distillates (FADs) from coconut oil by *Candida rugosa* until complete hydrolyzation. The disclosure of S. Nandi et al., Journal of Oleo Science Vol. 53(10) (2004), 497-501 is similar to that in the article in Enzyme and Microbial Technology 36. The starting Coconut Acid Oil (a by-product of coconut oil refinery industry) comprises 4.3 % monoglycerides (not described in more detail) and a ratio of lauric acid:caprylic acid of 50.3:4.9 = 10.27:1. The coconut acid oil thereof is almost completely hydrolyzed.

US 5 208 257 A discloses ethoxylated and propoxylated glycerol fatty acid esters. US 5 378 731 A describes shampoo compositions and similar compositions for the treatment of hair that involve the use of a fatty acid monoester of a polyhydroxy alcohol, such as monolaurin, as an antimicrobial agent. US 6,699,907 B1 discloses an antimocrobial composition comprising a mixture of C₈ to C₁₄ fatty acids. None of those three patents disclose any lipase-treated coconut oil.

EP 1 588 631 A1 discloses a lipase treated matter of oil and fat. Specific examples for the fat are salt-free butter, and for the oil coconut oil. This lipase treated matter is directly used for preventing growth of bacteria, yeast and fungi in food and drinks.

It seems that the ability of these medium chain fatty acids and their monoglycerides to act as antimicrobial agents is varied. Effectiveness of these compounds is always depending on their concentrations and the type of microbes involved. So far, antimicrobial activity of medium chain fatty acids and their corresponding monoglycerides was tested individually. Test was performed either using lauric acid and/or capric acid and/or caprylic acid and/or their corresponding monoglycerides. The synergistic effect of both medium chain fatty acids and their respective monoglycerides in oil medium has never been conducted and reported.

### Summary of the Invention

It is therefore an object of the invention to provide for modified coconut oil derived from catalyzing coconut oil with 1,3 -specific lipase as defined in claim 1, said modified coconut oil contains effective amount of medium-chain fatty-acids and their corresponding monoglycerides, wherein said modified coconut oil has antimicrobial properties. Said modified coconut oil has medium-chain fatty-acids comprise of caprylic acid (C₈), capric acid (C₁₀) and lauric acid (C₁₂). A further object of the invention are the uses of the coconut oil as defined in claims 2-9.

### Detailed Description of the Invention

Accordinlgy, efforts have been done in this work to get profiles of modified coconut oil that contains medium chain fatty acids (special emphasis on C₈-C₁₂) and their respective monoglycerides which have antimicrobial activities towards bacteria, yeast, fungi and viruses. Coconut oil was selected because by nature it was a rich source of medium chain fatty acids. It contained 90% saturated fatty acids, and of these fatty acids, 45-48% was lauric acid and 30-36% was other short- and medium chain fatty acids. Preliminary work showed that coconut oil in the present form did not have antimicrobial activities (it contained 7% diacylglycerol, less than 5% free fatty acids and the remainder was triglycerides). Analysis by HPTLC showed that little amount of fatty acids and monoglycerides were detected in the oil compositions. The invention also includes methods using 1,3 positional specific lipase to obtain not only oils that have antimicrobial properties but also effective and powerful antimicrobial agents.

According to the present invention 1,3 positional specific lipases is used to modify coconut oil under specific reaction conditions as described in Reaction 2 - Modified 3 and Reaction 5 -Modified 6 to obtain profiles of saturated medium chain free fatty acids and their respective monoglycerides that have broad antimicrobial spectrum towards bacteria and yeast. The modified coconut oil mixtures contain a high amount of free fatty acids (C₈-C₁₂) and their corresponding monoglycerides which can be obtained through partial hydrolysis and glycerolysis reaction. The enzyme used in the present invention is an enzyme such as lipase, and preferably immobilized onto a suitable enzyme carrier. The said specific lipase possess 1,3 -position i.e. Lipozyme TL IM (*Rhizomucor miehei*). Reactions to obtain modified oils were as followed.

### Reaction 1- Modified 1 and Modified 2 (for reference)

Enzymatic reaction was carried out using 2.5g 1,3- positional specific lipases with 250g coconut oil and 2.5 ml distilled water. The reaction was conducted at 45 °C at 250 r.p.m. Samples were withdrawn for analysis after 24 h reaction (Modified 1) and 120h reaction (Modified 2). Samples were then passed through a funnel containing sodium sulfate powder to remove water from the sample. The reaction mixture was centrifuged to separate the oil phase. High performance thin layer chromatography technique and gas chromatography technique were carried out to determine the lipid classes and fatty acids compositions of the oil sample respectively. The modified oils were then analyzed for their antimicrobial activities by Test 1: Minimal Microcidal Concentration (MMC, > 90%) and Test 2: Time-kill studies.

### Reaction 2- Modified 3

Twenty ml of coconut oil was added into a 125 ml flask containing 8 g glycerol and 160ul of distilled water. The reaction mixtures were incubated at 35°C, 300 r.p.m for a time until the incubation temperature reached 35°C. Lipozyme TL IM at 250 mg was then added into the reaction mixture and incubated for 24h at 35°C. The oil sample was then subjected for further incubation at 25°C for another 3days.

### Reaction 3- Modified 4 (for reference)

A mixture of 30.4g glycerol, 1.09 ml of water, 1g of Lipozyme TL IM and 100g of coconut oil was prepared. The reaction mixture was first incubated at 30°C for 6 h with constant stirring at 800 r.p.m. The mixture was then transferred to 5°C for up to 3 days before analysis on antimicrobial activities was performed.

### Reaction 4- Modified 5 (for reference)

The reaction mixture was prepared according to Modified 4. The reaction was carried out at 30°C, 800 r.p.m. for 16 h. before analysis on antimicrobial activities was performed.

### Reaction 5- Modified 6

Twenty ml oil from Modified 2 was added into a mixture containing 8 g glycerol, and 160µl sterile distilled water. The reaction mixture was pre incubated at 35°C, 300 r.p.m. The reaction was initiated by adding 250 mg Lipozyme TL IM into the mixture and reaction was carried for 24h. The mixture was then incubated at 25°C for 3 days.

### Test 1: Minimal Microcidal Concentration (MMC, > 90% kill)

All wells were inoculated with 120µl broth (BHI broth which contained 0.1% Tween 80 for Gram-positive bacteria; TSB broth for Gram-negative bacteria; PDB broth for yeast). 120µl of antimicrobial agent was inoculated into the first well. From said first well, 120µl of the mixture was transferred into the second well and so on until the 12^{th} well. Inoculum which was adjusted to 10⁵-10⁶ cfu/ml was inoculated into each well. The plates were incubated at 37°C (2 days) for bacteria and 32°C (3 days) for yeast. Results were expressed in terms of MMC₉₀ (minimal bactericidal concentration, >90% killing) as shown in Table 1.

### Test 2: Time-kill studies

Inocula were developed by inoculation of a loopful cell in 50ml broth (BHI fir Gram-positive bacteria; TSB for Gram-negative bacteria; PDB for yeast) in flask and shaked at optimum temperature overnight. This was used to inoculate BHI, TSB or PDB broth that contained 50% of filtered sterilized treated VCO. The initial inoculum was adjusted to 10⁴ - 10⁶ cfu/ml. At time interval, 1ml of reaction mixture was withdrawn and serial dilution was carried out in ringer solution. Viable colony was enumerated by plating dilutions on plate count agar (PCA). The plates were incubated at 37°C (2 days) for bacteria and 32°C (3 days) for yeast. A control experiment was done in the presence of 50% sterile distilled water.

**Table 1: Minimum Microcidal Concentration (MMC₉₀) of modified virgin coconut oils against pathogenic microorganisms**

| | Strain | | | | | |
|---|---|---|---|---|---|---|
| MBC, mg/ml | *S. aureus* | *L. monocytogenes* | *C .albicans* | *E. coli* | *S. pyogenes* | *P. acne* |
| Untreated Virgin Coconut Oil | none | none | none | none | none | none |
| Modified 1* | 156.25 | 156.25 | 156.25 | none | 156.25 | none |
| Modified 2* | 78.13 | 78.13 | 78.13 | none | 78.13 | none |
| Modified 3 | 4.88 | 4.88 | 2.44 | 2500 | 4.88 | none |
| Modified 4* | 78.13 | 78.13 | 2.44 | 2500 | 78.13 | none |
| Modified 5* | 78.13 | 78.13 | 4.88 | 2500 | 78.13 | none |
| Modified 6 | 4.88 | 4.88 | 2.44 | 2500 | 4.88 | none |

| | | | | | | |
|---|---|---|---|---|---|---|
| *: for reference | | | | | | |

**Table 2: Inhibition of pathogenic microorganisms by modified virgin coconut oils evaluated by time-kill studies**

| Microbes | Modified Virgin Coconut Oil | Number of viable bacteria (log10 cfu/ml) at time interval (hours) | | | | | | |
|---|---|---|---|---|---|---|---|---|
| | | 0 | 2 | 4 | 6 | 8 | 24 | 48 |
| *S*. *aureus* | Modified 1* | 5.69 | 5 | 3.6 | 3.11 | 2.62 | 0 | 0 |
| | Modified 2* | 6.32 | 0 | 0 | 0 | 0 | 0 | 0 |
| | Modified 3 | 6.27 | 0 | 0 | 0 | 0 | 0 | 0 |
| | Modified 4* | 6.04 | 4.25 | 0 | 0 | 0 | 0 | 0 |
| | Modified 5* | 6.08 | 3.96 | 0 | 0 | 0 | 0 | 0 |
| | Modified 6 | 5.87 | 0 | 0 | 0 | 0 | 0 | 0 |
| *L. monocytogenes* | Modified 1* | 5.71 | 5.02 | 3.62 | 3.15 | 2.66 | 0 | 0 |
| | Modified 2* | 6.28 | 0 | 0 | 0 | 0 | 0 | 0 |
| | Modified 3 | 6.15 | 0 | 0 | 0 | 0 | 0 | 0 |
| | Modified 4* | 6.06 | 4.25 | 0 | 0 | 0 | 0 | 0 |
| | Modified 5* | 6.08 | 3.76 | 0 | 0 | 0 | 0 | 0 |
| | Modified 6 | 5.34 | 0 | 0 | 0 | 0 | 0 | 0 |
| C. albicans | Modified 1* | 4.59 | 4.61 | 4.54 | 4.44 | 4.29 | 2.81 | 2.71 |
| | Modified 2* | 6.48 | 5.56 | 5.48 | 5.65 | 5.38 | 2.16 | 2 |
| | Modified 3 | 5.76 | 4.98 | 3.21 | 3.45 | 3.11 | 0 | 0 |
| | Modified 4* | 5.98 | 5.65 | 5.15 | 4.18 | 3.46 | 0 | 0 |
| | Modified 5* | 6.02 | 5.68 | 5.43 | 4.75 | 3.86 | 0 | 0 |
| | Modified 6 | 5.98 | 4.13 | 3.06 | 2.99 | 2.14 | 0 | 0 |
| *E. coli* | Modified 1* | ND | ND | ND | ND | ND | ND | ND |
| | Modified 2* | 6.32 | 6.48 | 7.52 | 8.34 | 9.16 | 10.27 | 11.16 |
| | Modified 3 | 6.21 | 6.54 | 5.82 | 5.96 | 4.32 | 2.58 | 2.69 |
| | Modified 4* | 6.01 | 6.26 | 5.64 | 5.28 | 5.04 | 2.96 | 2.71 |
| | Modified 5* | 6.02 | 6.15 | 6.01 | 5.78 | 5.44 | 2.84 | 2.56 |
| | Modified 6 | 6.14 | 5.28 | 5.35 | 6.02 | 4.67 | 2.64 | 2.63 |
| S. pyogenes | Modified 1* | 5.48 | 5.21 | 4.23 | 3.08 | 2.12 | 0 | 0 |
| | Modified2* | 6.1 | 0 | 0 | 0 | 0 | 0 | 0 |
| | Modified 3 | 5.7 | 0 | 0 | 0 | 0 | 0 | 0 |
| | Modified 4* | 6.01 | 4.68 | 0 | 0 | 0 | 0 | 0 |
| | Modified 5* | 6.04 | 4.74 | 0 | 0 | 0 | 0 | 0 |
| | Modified 6 | 5.49 | 0 | 0 | 0 | 0 | 0 | 0 |
| *V*. *cholerae* | Modified2* | 6.34 | 0 | 0 | 0 | 0 | 0 | 0 |

| | | | | | | | | |
|---|---|---|---|---|---|---|---|---|
| (*: for reference) | | | | | | | | |

**Table 3: Inhibition of pathogenic microorganisms by modified palm kernel oil evaluated by kill studies**

| Microbe | Number of viable bacteria (log cfu/ml) at time interval (hours) | | | | | | |
|---|---|---|---|---|---|---|---|
| | 0 | 2 | 4 | 6 | 8 | 24 | 48 |
| *S. aureus* | 7.28 | 7.24 | 7.18 | 7.18 | 5.31 | 5.85 | 0 |
| *E. coli* | 6.93 | 7.30 | 10.76 | 12.10 | 13.56 | 10.22 | 8.56 |
| *C. albicans* | 5.71 | 6.03 | 5.92 | 5.95 | 5.87 | 5.98 | 5.74 |

The following profiles of modified virgin coconut oils and palm kernel oil have been tested for their antimicrobial activities and found to be active against a substantial group of microorganisms.

**MODFIED 1- Profile 1 (for reference)**

| Lipid Classes | Percentages (% area by HPTLC) | Medium chain length | Concentration (mg/g) |
|---|---|---|---|
| Fatty acids | 10.58 | Caprylic acid (C₈) | 10.44 |
| | | Capric acid (C₁₀) | 7.27 |
| | | Lauric acid (C₁₂) | 54.61 |
| Monoglycerides | 1.32 | Monocaprylin | 0.67 |
| | | Monocaprin | 0.42 |
| | | Monolaurin | 20.96 |
| Diglycerides | 22.81 | | |
| Triglycerides | 65.29 | | |

**MODIFIED 2- Profile 2 (for reference)**

| Lipid classes | Percentages (% area by HPTLC) | Medium chain length | Concentration (mg/g) |
|---|---|---|---|
| Fatty acids | 14.13 | Caprylic acid (C₈) | 13.95 |
| | | Capric acid (C₁₀) | 9.89 |
| | | Lauric acid (C₁₂) | 70.54 |
| Monoglycerides | 1.51 | Monocaprylin | 1.30 |
| | | Monocaprin | 0.50 |
| | | Monolaurin | 27.00 |
| Diglycerides | 26.88 | | |
| Triglycerides | 57.48 | | |

**MODIFIED 3-Profile 3**

| Lipid Classes | Percentages (% area by HPTLC) | Medium chain length | Concentration (mg/g) |
|---|---|---|---|
| Fatty acids | 23.40 | Caprylic acid (C8) | 23.10 |
| | | Capric acid (C10) | 16.08 |
| | | Lauric acid (C12) | 116.81 |
| Monoglycerides | 14.28 | Monocaprylin | 16.04 |
| | | Monocaprin | 10.35 |
| | | Monolaurin | 75.54 |
| Diglycerides | 37.24 | | |
| Triglycerides | 25.08 | | |

**MODIFIED 4- Profile 4 (for reference)**

| Lipid Classes | Percentages (% by HPTLC) | Medium chain length | Concentration (mg/g) |
|---|---|---|---|
| Fatty acids | 13.44 | Caprylic acid (C₈) | 13.35 |
| | | Capric acid (C₁₀) | 9.34 |
| | | Lauric acid (C₁₂) | 68.61 |
| Monoglycerides | 8.04 | Monocaprylin | 8.00 |
| | | Monocaprin | 5.40 |
| | | Monolaurin | 41.10 |
| Diglycerides | 31.36 | | |
| Triglycerides | 47.16 | | |

**MODIFIED 5- Profile 5 (for reference)**

| Lipid Classes | Percentages (% area by HPTLC) | Medium chain length | Concentration (mg/g) |
|---|---|---|---|
| Fatty acids | 9.40 | Caprylic acid (C₈) | 9.40 |
| | | Capric acid (C₁₀) | 6.56 |
| | | Lauric acid (C₁₂) | 71.77 |
| Monoglycerides | 12.10 | Monocaprylin | 12.25 |
| | | Monocaprin | 8.05 |
| | | Monolaurin | 71.77 |
| Diglycerides | 39.98 | | |
| Triglycerides | 38.47 | | |

**MODIFIED 6- Profile 6**

| Lipid Classes | Percentages (% area by HPTLC) | Medium chain length | Concentration (mg/g) |
|---|---|---|---|
| Fatty acids | 25.01 | Caprylic acid (C₈) | 24.63 |
| | | Capric acid (C₁₀) | 17.81 |
| | | Lauric acid (C₁₂) | 133.70 |
| Monoglycerides | 11.45 | Monocaprylin | 11.82 |
| | | Monocaprin | 8.29 |
| | | Monolaurin | 57.16 |
| Diglycerides | 35.98 | | |
| Triglycerides | 27.56 | | |

**Modified Palm Kernel Oil (for reference)**

| Lipid Classes | Percentages (% area by HPTLC) | Medium chain length | Concentration (mg/g) |
|---|---|---|---|
| Fatty acids | 12.87 | Caprylic acid (C₈) | 16.10 |
| | | Capric acid (C₁₀) | 13.06 |
| | | Lauric acid (C₁₂) | 58.19 |
| Monoglycerides | 1.31 | Monocaprylin | 1.64 |
| | | Monocaprin | 2.03 |
| | | Monolaurin | 19.71 |
| Diglycerides | 21.66 | | |
| Triglycerides | 64.16 | | |

According to the invention, the methods according to Modified 3 and Modified 6 to obtain antimicrobial compositions containing i.a. medium chain free fatty acids and their corresponding monoester through partial hydrolysis and glyecrolysis of coconut oil are provided. Modified oil 1 and Modified oil 2 were obtained from hydrolysis reaction of virgin coconut oil at 24 h and 120h, respectively. Test for antimicrobial activities as MMC₉₀ (Table 1) and time-kill studies (Table 2) showed that Modified 2 has more powerful antimicrobial activities compared to Modified 1. This was probably because of high amount of fatty acids (14%) present in Modified 2 (Profile 2). The amount of saturated medium chain fatty acids especially caprylic, capric and lauric (mg/g) oil were increased (Profile 2). However, it was noticed that modified oil compositions from Profile 1 and Profile 2 can't stop the growth of *E .coli* and *P. acne.* Obviously, from the time-kill studies C. *albicans* growth was not 100% inhibited even after 48h incubation (Table 2). Modified 1 and Modified 2 were effective toward gram positive bacteria where 100% inhibitions were noted after 8 h and 2 h incubation, respectively. Preliminary results indicated that the amount fatty acids i.e. C₈, C₁₀ and C₁₂ might play important role in inactivation of the growth of gram positive bacteria. Modified 3, 4, 5 and 6 were prepared differently from Modified 1 and 2. They were obtained through hydrolysis followed by glycerolysis reactions. In all reactions glycerol and Lipozyme TL IM were added.

In comparison, Modified 3, 4, 5 and 6 were more effective in killing *C. albicans* than Modified 1 and 2. Hundred percent inhibitions were noted after 8 hours incubation. Minimal Microbial Concentration towards *C. albicans* was obtained at 2.44 mg/ml whereas Modified 5 needed higher concentration e.g. 4.88 mg/ml. Among the modified oil samples, Modified 3 and Modified 6 contained powerful antimicrobial activities. In addition it was noted that MMC₉₀ for gram positive bacteria like *S. aureus, L. monocytogenes, S*. *pyogenes* of Modified 3 according to the invention , 4, 5 and 6 was lower than MMC₉₀ in Modified 1 and 2. The most interesting thing was that these modified coconut oils have antimicrobial activity towards gram negative, *E. coli* although the MMC₉₀ required was still higher compared to the gram positive bacteria. Modified 3 and 6 were also proved to be more potent toward gram positive bacteria compared to Modified 4 and 5. Detail analysis of the lipid classes of Modified 3, 4, 5 and 6 showed that high amount of fatty acids and monoglycerides content played an important role in broaden the antimicrobial spectrum of these modified oils. About 8% to 14% monoglycerides were required to control and reduce the growth of *E. coli* and 100% inhibition of *C. albicans.* Whereas high amount of fatty acids in Modified 3 and 6 made the modified oils more potent towards gram positive bacteria (Profile 3 and Profile 6). The MMC₉₀ for all gram positive bacteria for Modified 3 and 6 was at 4.88 mg/ml which was sixteen times lower than that of Modified 4 and 5. Fatty acids together with monoglycerides have a synergistic effect that inhibited 100% growth of *C. albicans.*

Palm kernel oil, whose fatty acid composition was similar with coconut oil, can also be modified using 1,3 specific lipase. The modified palm kernel oil was also found to have antimicrobial property towards *S. aureus.* Total inhibition of *S*. *aureus* was noted after 24 h exposure. On the other hand it was noted that the amount of medium chain fatty acids and their corresponding fatty acids in the modified palm kernel oil was not significant to stop the growth or kill the *E. coli* and *C. albicans*

The modified oils compositions of the present invention were found to exhibit good shelf stability against oxidation and can be safely used to combat bacteria, yeast and viruses that affect human, as food preservatives, products for personal hygiene and prevention of skin infection.

The scope of the invention is to be construed in accordance with the substance defined by the following claims.

## Claims

1. A modified virgin coconut oil derived from catalyzing coconut oil with 1,3-specific lipase and subjecting it to glycerolysis, said modified coconut oil containing 24.63 mg/g caprylic acid (C8), 17.81 mg/g capric acid (C10), 133.70 mg/g lauric acid (C12) and 11.82 mg/g monocaprylin, 8.29 mg/g monocaprin and 57.16 mg/g monolaurin; or 23.10 mg/g caprylic acid (C8), 16.08 mg/g capric acid (C10), 116.81 mg/g lauric acid (C12) and 16.04 mg/g monocaprylin, 10.35 mg/g monocaprin and 75.54 mg/g monolaurin.

2. The modified virgin coconut oil of claim 1 for use in combating gram-positive bacteria that affect humans.

3. The modified virgin coconut oil of claim 1 for use in preventing skin infections caused by gram-positive bacteria.

4. The modified virgin coconut oil of claim 1 for use according to claim 2 or 3, wherein the gram-positive bacteria are selected from *S. aureus, L. monocytogenenes* and *S. pyogenes.*

5. Non-therapeutic use of the modified virgin coconut oil of claim 1 for inhibiting the growth of gram-positive bacteria.

6. The non-therapeutic use according to claim 5, wherein the gram-positive bacteria are selected from *S. aureus, L. monocytogenenes* and *S. pyogenes.*

7. The modified virgin coconut oil of claim 1 for use in combating the yeast *C. albicans* that affects humans.

8. The modified virgin coconut oil of claim 1 for use in preventing skin infections caused by the yeast *C. albicans.*

9. Non-therapeutic use of the modified virgin coconut oil of claim 1 for inhibiting the growth of the yeast *C. albicans.*

## Patentansprüche

1. Ein modifiziertes natives Kokosnussöl erhalten durch eine Katalyse von Kokosnussöl mit 1,3-spezifischer Lipase und dessen Unterziehung einer Glycerolyse, wobei das native Kokosnussöl 24.63 mg/g Caprylsäure (C8), 17.81 mg/g Caprinsäure (C10), 133.70 mg/g Laurinsäure (C12) und 11.82 mg/g Monocaprylin, 8.29 mg/g Monocaprin und 57.16 mg/g Monolaurin; oder 23.10 mg/g Caprylsäure (C8), 16.08 mg/g Caprinsäure (C10), 116.81 mg/g Laurinsäure (C12) und 16.04 mg/g Monocaprylin, 10.35 mg/g Monocaprin und 75.54 mg/g Monolaurin enthält.

2. Das modifizierte native Kokosnussöl nach Anspruch 1, zur Verwendung in der Bekämpfung von gram-positiven Bakterien, welche den Menschen befallen.

3. Das modifizierte native Kokosnussöl nach Anspruch 1, zur Verwendung in der Verhinderung von Hautinfektionen, welche von gram-positiven Bakterien verursacht werden.

4. Das modifizierte native Kokosnussöl nach Anspruch 1 zur Verwendung gemäß Anspruch 2 oder 3, wobei die gram-positiven Bakterien aus *S*. *aureus, L. monocytogenenes* und *S*. *pyogenes* ausgewählt sind.

5. Nicht-therapeutische Verwendung des modifizierten nativen Kokosnussöls nach Anspruch 1, zur Inhibierung des Wachstums gram-positiver Bakterien.

6. Die nicht-therapeutische Verwendung nach Anspruch 5, wobei die gram-positiven Bakterien aus *S*. *aureus, L. monocytogenenes* und *S. pyogenes* ausgewählt sind.

7. Das modifizierte native Kokosnussöl nach Anspruch 1, zur Verwendung bei der Bekämpfung der Hefe *C. albicans,* die den Menschen befällt.

8. Das modifizierte native Kokosnussöl nach Anspruch 1, zur Verwendung bei der Verhinderung von Hautinfektionen, welche durch die Hefe *C. albicans* verursacht werden.

9. Die nicht-therapeutische Verwendung des modifizierten nativen Kokosnussöls nach Anspruch 1, zur Inhibierung des Wachstums der Hefe *C. albicans.*

## Revendications

1. Huile de noix de coco vierge modifiée dérivée à partir de la catalyse de l'huile de noix de coco avec une lipase 1,3-spécifique et de sa soumission à une glycérolyse, ladite huile de noix de coco modifiée contenant 24,63 mg/g d'acide caprylique (C8), 17,81 mg/g d'acide caprique (C10), 133,70 mg/g d'acide laurique (C12) et 11,82 mg/g de monocapryline, 8,29 mg/g de monocaprine et 57,16 mg/g de monolaurine ; ou 23,10 mg/g d'acide caprylique (C8), 16,08 mg/g d'acide caprique (C10), 116,81 mg/g d'acide laurique (C12) et 16,04 mg/g de monocapryline, 10,35 mg/g de monocaprine et 75,54 mg/g de monolaurine.

2. Huile de noix de coco vierge modifiée selon la revendication 1 pour une utilisation dans le combat des bactéries gram-positives qui affectent les êtres humains.

3. Huile de noix de coco vierge modifiée selon la revendication 1 pour une utilisation dans la prévention des infections de la peau provoquées par des bactéries gram-positives.

4. Huile de noix de coco vierge modifiée selon la revendication 1 pour une utilisation selon la revendication 2 ou 3, dans lequel les bactéries gram-positives sont sélectionnées parmi *S. aureus, L. monocytogenenes* et *S*. *pyogenes.*

5. Utilisation non thérapeutique de l'huile de noix de coco vierge modifiée selon la revendication 1 pour inhiber la croissance de bactéries gram-positives.

6. Utilisation non thérapeutique selon la revendication 5, dans laquelle les bactéries gram-positives sont sélectionnées parmi *S*. *aureus, L. monocytogenenes* et *S*. *pyogenes.*

7. Huile de noix de coco vierge modifiée selon la revendication 1 pour une utilisation dans le combat de la levure *C. albicans* qui affecte les êtres humains.

8. Huile de noix de coco vierge modifiée selon la revendication 1 pour une utilisation dans la prévention des infections de la peau provoquées par la levure *C. albicans.*

9. Utilisation non thérapeutique de l'huile de noix de coco vierge modifiée selon la revendication 1 pour inhiber la croissance de la levure *C. albicans.*
